# FASCICULE DE BREVET EUROPEEN

(11) **EP 1 116 497 B1**
(45) Date de publication et mention de la délivrance du brevet: **21.09.2005**
(21) Numéro de dépôt: 01400086.3
(22) Date de dépôt: 12.01.2001
(51) Int. Cl.: A61N 1/368, A61N 1/365

(54) **Dispositif médical implantable actif, notamment stimulateur cardiaque, défibrillateur et/ou dispositif multisite comportant des moyens de mesure d'impédance transvalvulaire**
Aktive implantierbare medizinische Vorrichtung, insbesondere Herzstimulator, Defibrillator und/oder Multisite-Vorrichtung mit Mitteln zur Messung der transvalvularen Impedanz
Active implantable medical device, in particular pacemaker, defibrillator and/or multisite device having means to measure transvalvular impedance

(30) Priorité: 14.01.2000 FR 0000482
(43) Date de publication de la demande: 18.07.2001
(73) Titulaire: ELA MEDICAL, F-92541 Montrouge (FR)
(72) Inventeur: Dal Molin, Renzo, 92320 Chatillon (FR)
(74) Mandataire: Dupuis-Latour, Dominique

(56) Documents cités:
- WO-A-99/30777
- US-A- 5 501 702

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle peut avantageusement concerner, plus particulièrement, les prothèses dites "multisite" dans lesquelles des électrodes sont placées en une pluralité de sites respectifs distincts comportant au moins un site ventriculaire et un site auriculaire. Il peut s'agir d'une prothèse de type "double chambre" (stimulation atriale droite et stimulation ventriculaire droite) ou, le plus souvent, "triple chambre" (stimulation atriale droite et double stimulation ventriculaire) ou "quadruple chambre" (double stimulation atriale et double stimulation ventriculaire).

Le pilotage de la stimulation implique l'ajustement permanent de divers paramètres tels que fréquence de stimulation, délai atrioventriculaire (DAV), ou encore délai interventriculaire dans le cas d'une stimulation biventriculaire.

Ces divers paramètres sont ajustés en fonction de signaux délivrés par des capteurs, par exemple de la ventilation-minute (MV), qui est un facteur représentatif des besoins métaboliques instantanés du patient.

Un autre facteur qu'il peut être intéressant de connaître est le débit cardiaque car il peut être intéressant, tout particulièrement avec un stimulateur multisite, d'obtenir une indication de ce débit et donc de la fraction d'éjection, qui est le paramètre hémodynamique de référence pour optimiser la stimulation sur les différents sites.

Le WO-A-99/34863 (Pacesetter AB) décrit une sonde permettant d'obtenir une indication de ce facteur par mesure de la pression intracardiaque grâce à un capteur piézoélectrique incorporé à l'extrémité d'une sonde ; la pression est intégrée entre l'instant d'ouverture et l'instant de fermeture de la valvule, ce qui donne une indication du travail fourni par le muscle cardiaque, information qui est utilisée pour, entre autres, piloter la fréquence de stimulation et le délai atrioventriculaire.

Cette manière de procéder, si elle est efficace, nécessite cependant une sonde spécifique incorporant le capteur piézoélectrique, ainsi qu'une électronique particulière pour exploiter les signaux issus de ce capteur, les convertir et les transmettre au microprocesseur du stimulateur.

Un autre paramètre corrélé au débit cardiaque est l'impédance transvalvulaire, généralement mesurée sur le coeur droit.

À cet égard, le US-A-5 154 171 (Chirife) décrit la manière d'effectuer une mesure dynamique de bioimpédance permettant d'évaluer les volumes diastoliques et systoliques, et d'obtenir ainsi une indication du débit cardiaque et donc de la fraction d'éjection. Le signal obtenu est utilisé pour piloter la fréquence cardiaque dans le sens de la maximisation du débit.

Ce document propose d'effectuer la mesure de bioimpédance par injection d'une impulsion de courant entre deux points et recueil d'un potentiel différentiel entre ces deux mêmes points.

En pratique, on constate cependant que cette configuration d'injection/recueil se révèle sensible au mouvement des sondes et ne permet pas une mesure fiable et précise de l'impédance transvalvulaire.

L'un des buts de l'invention est de remédier à cet inconvénient en proposant une nouvelle configuration de mesure de l'impédance transvalvulaire avec un choix particulier des sites d'injection et de recueil.

Un autre but de l'invention est d'utiliser le paramètre ainsi mesuré pour piloter le délai interventriculaire, dans le cas d'une stimulation biventriculaire (l'impédance transvalvulaire pouvant bien entendu être également utilisée dans tous les cas pour piloter la fréquence de stimulation et/ou le délai atrioventriculaire).

Plus précisément, l'invention concerne un dispositif médical implantable actif, notamment un stimulateur cardiaque, défibrillateur et/ou cardioverteur et/ou dispositif multisite dans lequel des électrodes sont placées en une pluralité de sites respectifs distincts comportant au moins un site ventriculaire et un site auriculaire d'un même côté du coeur, ces électrodes étant reliées à des moyens de recueil de signaux cardiaques, pour détecter un potentiel de dépolarisation, ainsi qu'à des moyens de stimulation, pour appliquer des impulsions de stimulation sur au moins certains desdits sites. Ce dispositif comporte en outre des moyens d'évaluation du débit cardiaque par mesure de bioimpédance transvalvulaire, ces moyens opérant par injection d'un courant entre un site atrial et un site ventriculaire et recueil d'un potentiel différentiel entre un site atrial et un site ventriculaire.

Selon l'invention, la configuration de mesure de bioimpédance transvalvulaire est une configuration tripolaire, avec un site commun à l'injection et au recueil, un site propre d'injection et un site propre de recueil, le site commun étant situé dans l'une des cavités et les deux sites propres étant situés dans l'autre cavité.

Le dispositif peut comporter en outre des moyens pour faire varier le délai inter-ventriculaire d'application des impulsions de stimulation respectives des ventricules droit et gauche (dans le cas d'un dispositif multisite), et/ou des moyens pour faire varier la fréquence d'application des impulsions de stimulation, et/ou des moyens pour faire varier le délai atrio-ventriculaire d'application des impulsions de stimulation. Ces divers moyens opèrent en réponse à l'impédance transvalvulaire mesurée dans le sens de l'amélioration du débit cardiaque.

Selon diverses caractéristiques subsidiaires avantageuses :
- le site commun est un site auriculaire et les deux sites propres sont des sites ventriculaire ;
- le site commun est un site ventriculaire et les deux sites propres sont des sites auriculaires ;
- le site commun est une électrode proximale d'une sonde ;
- les deux sites propres sont une électrode proximale et une électrode distale d'une même sonde.
- dans une configuration préférentielle, le site commun à l'injection et au recueil est une électrode proximale d'une sonde auriculaire droite, le site propre à l'injection est une électrode distale d'une sonde ventriculaire droite et le site propre de recueil est une électrode proximale de cette même sonde ventriculaire droite.

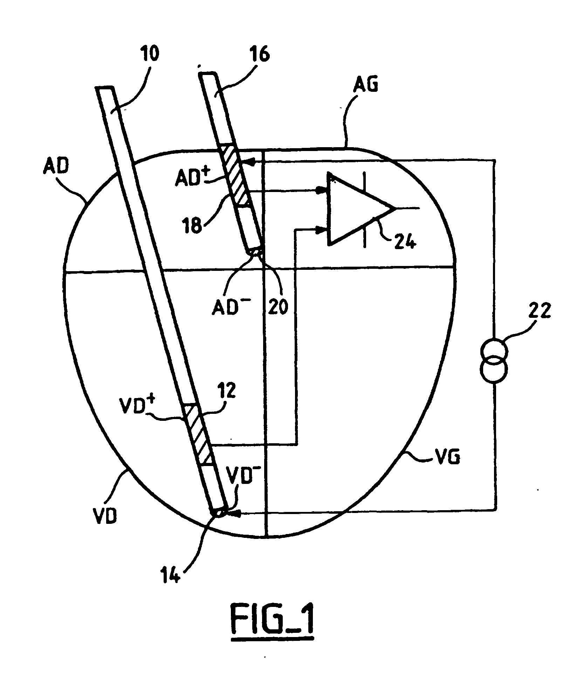

On va maintenant décrire un exemple de réalisation de l'invention, en référence au dessin annexé, où la figure 1 est une vue schématique d'un muscle cardiaque avec différents sites de stimulation.

La figure 1 représente schématiquement un muscle cardiaque avec ses quatre cavités : oreillette droite AD, oreillette gauche AG, ventricule droit VD, ventricule gauche VG.

Une sonde 10 est introduite dans le ventricule droit VD, avec une électrode proximale annulaire 12, référencée VD+, et une électrode distale d'extrémité 14, référencée VD-.

Une sonde 16 est introduite dans l'oreillette droite AD, avec une électrode proximale annulaire 18, référencée AD+, et une électrode distale d'extrémité 20, référencée AD-.

Le cas échéant, il peut également être prévu une sonde sur le ventricule gauche VG, par exemple pour permettre une stimulation biventriculaire (configuration triple chambre) et/ou une sonde sur l'oreillette gauche AG, si l'on souhaite réaliser un recueil de signaux et/ou une stimulation sur les deux oreillettes (configuration quadruple chambre).

Les électrodes des sondes sont reliées à un boîtier comprenant divers circuits de détection, de stimulation et de commande, par exemple un boîtier de stimulateur multisite tel que celui décrit dans le EP-A-0 925 806 (ELA Médical) auquel on pourra se référer pour de plus amples détails.

Parmi ces circuits, il est prévu un circuit de mesure de bioimpédance transvalvulaire (c'est-à-dire entre l'oreillette droite AD et le ventricule droit VD), mesure réalisée classiquement par injection d'une impulsion de courant (schématisée par le générateur de courant 22) entre deux points, et recueil d'un potentiel différentiel (schématisé par l'amplificateur opérationnel 24) entre deux points.

L'injection et le recueil des signaux en ces différents points est réalisé par un circuit tel que celui décrit dans le US-A-5 154 171 précité, ou encore par un circuit tel que celui servant à la mesure de la ventilation-minute (MV) sur les dispositifs existants.

Dans ce dernier cas, les sites sont modifiés : injection/recueil intracardiaques, et non injection/recueil transpulmonaires (entre coeur et boîtier). Par ailleurs, le recueil est opéré dans des bandes de fréquences différentes : basse fréquence pour la mesure de MV, fréquence plus élevée pour la mesure d'impédance transvalvulaire selon l'invention.

En d'autres termes, il est possible de réutiliser la chaîne de mesure de ventilation-minute classique préexistante du dispositif, en récupérant le signal de mesure avant l'étage de filtrage MV, permettant donc une mise en oeuvre de l'invention sans surcoût important.

Le courant injecté pour la mesure d'impédance est par exemple un courant de 40 µA délivré sous la forme d'une impulsion de largeur 5 µs.

Dans le document précité US-A-5 154 171, les points d'injection et de recueil sont les mêmes (configuration bipolaire), tandis que dans la présente invention la configuration de mesure est une configuration tripolaire, avec un point commun à l'injection et au recueil.

Diverses configurations d'injection et de recueil sont possibles.

La configuration illustrée, actuellement préférée, est :
- injection entre AD+ et VD-
- recueil entre AD+ et VD+.

En d'autres termes, le point commun, c'est-à-dire la référence de mesure, est l'électrode proximale auriculaire AD+.

Le signal ainsi recueilli peut être utilisé pour piloter la fréquence de stimulation et/ou le délai atrio-ventriculaire et/ou (et de façon caractéristique de l'invention) le délai interventriculaire dans le cas d'une stimulation biventriculaire. L'ajustement de ces divers paramètres se fait bien entendu dans le sens procurant le débit cardiaque maximal.

D'autres configurations tripolaires de mesure sont envisageables.

Il est ainsi possible d'inverser les rôles des sites VD+ et VD-, c'est-à-dire :
- injection entre AD+ et VD+
- recueil entre AD+ et VD-.

De même, il est possible de choisir AD- au lieu de AD+ comme site commun, c'est-à-dire :
- injection entre AD- et VD-
- recueil entre AD- et VD+,
ou encore :
- injection entre AD- et VD+
- recueil entre AD- et VD-.

Il est également possible d'inverser le rôle de l'oreillette et du ventricule, c'est-à-dire de choisir comme site commun un site ventriculaire. Si l'on choisit ainsi VD+ comme site commun, on pourra avoir :
- injection entre VD+ et AD-
- recueil entre VD+ et AD+.

Il est également possible, dans cette dernière configuration, de permuter les rôles de AD+ et AD-, ou encore de choisir VD- au lieu de VD+ comme site commun.

Par ailleurs, tout ce qui vient d'être dit pour le coeur droit (cavités AD et VD) est transposable au coeur gauche (cavités VG et AG), bien que ce choix entraîne une plus grande complexité et de moindres performances, du fait notamment du recours obligé à des sondes coronaires au lieu de sondes intracardiaques.

## Revendications

1. Un dispositif médical implantable actif, notamment un stimulateur cardiaque, défibrillateur et/ou cardioverteur et/ou dispositif multisite dans lequel des électrodes sont placées en une pluralité de sites respectifs distincts comportant au moins un site ventriculaire et un site auriculaire d'un même côté du coeur, ces électrodes étant reliées à des moyens de recueil de signaux cardiaques, pour détecter un potentiel de dépolarisation, ainsi qu'à des moyens de stimulation, pour appliquer des impulsions de stimulation sur au moins certains desdits sites,
ce dispositif comportant en outre des moyens d'évaluation du débit cardiaque par mesure de bioimpédance transvalvulaire, ces moyens opérant par injection d'un courant entre un site atrial et un site ventriculaire et recueil d'un potentiel différentiel entre un site atrial et un site ventriculaire, dispositif **caractérisé en ce que** la configuration de mesure de bioimpédance transvalvulaire est une configuration tripolaire, avec un site commun à l'injection et au recueil, un site propre d'injection et un site propre de recueil, le site commun (AD+) étant situé dans l'une des cavités et les deux sites propres (VD+, VD-) étant situés dans l'autre cavité.

2. Le dispositif de la revendication 1, ce dispositif étant un dispositif multisite comprenant en outre des moyens pour faire varier le délai inter-ventriculaire d'application des impulsions de stimulation respectives des ventricules droit et gauche, ces moyens opérant en réponse à l'impédance transvalvulaire mesurée dans le sens de l'amélioration du débit cardiaque.

3. Le dispositif de la revendication 1, comprenant en outre des moyens pour faire varier la fréquence d'application des impulsions de stimulation, ces moyens opérant en réponse à l'impédance transvalvulaire mesurée dans le sens de l'amélioration du débit cardiaque.

4. Le dispositif de la revendication 1, comprenant en outre des moyens pour faire varier le délai atrio-ventriculaire d'application des impulsions de stimulation, ces moyens opérant en réponse à l'impédance transvalvulaire mesurée dans le sens de l'amélioration du débit cardiaque.

5. Le dispositif de la revendication 1, dans lequel le site commun est un site d'électrode auriculaire (AD+ ; AD-) et les deux sites propres sont des sites d'électrodes ventriculaires (VD+, VD-; VD-, VD+).

6. Le dispositif de la revendication 1, dans lequel le site commun est un site d'électrode ventriculaire (VD+ ; VD-) et les deux sites propres sont des sites d'électrodes auriculaires (AD+, AD-; AD-, AD+).

7. Le dispositif de la revendication 1, dans lequel le site commun est une électrode proximale (18) d'une sonde (16).

8. Le dispositif de la revendication 1, dans lequel les deux sites propres sont une électrode proximale (12) et une électrode distale (14) d'une même sonde (10).

9. Le dispositif de la revendication 1, dans lequel le site commun à l'injection et au recueil est une électrode proximale (18) d'une sonde auriculaire droite (16), le site propre à l'injection est une électrode distale (14) d'une sonde ventriculaire droite (10) et le site propre de recueil est une électrode proximale (12) de cette même sonde ventriculaire droite (10).

## Patentansprüche

1. Medizinische implantierbare aktive Vorrichtung, insbesondere Herzschrittmacher, Defibrillator und/oder Kardioverter und/oder Multisite-Vorrichtung, in welcher Elektroden an eine Mehrzahl von jeweils unterschiedlichen Stellen gesetzt werden, welche mindestens eine ventrikuläre Stelle und eine aurikuläre Stelle einer gleichen Seite des Herzens umfassen, wobei diese Elektroden mit Aufnahmemitteln von Herzsignalen verbunden sind zum Detektieren eines Depolarisationspotentials sowie mit Stimulationsmitteln zum Anlegen von Stimulationsimpulsen auf mindestens einige der Stellen,
wobei diese Vorrichtung des Weiteren Auswertungsmittel des Herzdurchsatzes umfasst durch Messung der transvalvulären Bioimpedanz, wobei diese Mittel durch Injektion eines Stroms zwischen einer atrialen Stelle und einer ventrikulären Stelle arbeiten und durch Empfang eines Differenzpotentials zwischen einer atrialen Stelle und einer ventrikulären Stelle,
wobei die Vorrichtung **dadurch gekennzeichnet ist, dass** die Konfiguration der Messung der transvalvulären Bioimpedanz eine dreipolige Konfiguration ist mit einer der Injektion und dem Empfang gemeinsamen Stelle, einer der Injektion eigenen Stelle und einer dem Empfang eigenen Stelle,
wobei die gemeinsame Stelle (AD+) in einer der Vertiefungen angeordnet ist und die zwei eigenen Stellen (VD+, VD-) in der anderen Vertiefung angeordnet sind.

2. Vorrichtung nach Anspruch 1, wobei diese Vorrichtung eine Multisite-Vorrichtung ist, welche des Weiteren Mittel aufweist zum Variierenlassen der inter-ventrikulären Verzögerung einer Anlegung von jeweiligen Stimulationsimpulsen der rechten und linken Herzkammern, wobei diese Mittel in Antwort auf die transvalvuläre Impedanz betrieben werden, welche in Richtung der Verbesserung des Herzdurchsatzes gemessen wird.

3. Vorrichtung nach Anspruch 1, welche des Weiteren Mittel aufweist zum Variierenlassen der Frequenz einer Anlegung von Stimulationsimpulsen, wobei diese Mittel in Antwort auf die transvalvuläre Impedanz betrieben werden, welche in der Richtung der Verbesserung des Herzdurchsatzes gemessen wird.

4. Vorrichtung nach Anspruch 1, welche des Weiteren Mittel aufweist zum Variierenlassen der atrio-ventrikulären Verzögerung einer Anlegung von Stimulationsimpulsen, wobei diese Mittel in Antwort auf die transvalvuläre Impedanz betrieben werden, welche in der Richtung der Verbesserung des Herzdurchsatzes gemessen wird.

5. Vorrichtung nach Anspruch 1, in welcher die gemeinsame Stelle eine Stelle einer aurikularen Elektrode (AD+; AD-) ist und die zwei eigenen Stellen Stellen ventrikulärer Elektroden (VD+, VD-; VD-, VD+) sind.

6. Vorrichtung nach Anspruch 1, in welcher die gemeinsame Stelle eine Stelle einer ventrikulären Elektrode (VD+; VD-) ist und die zwei eigenen Stellen Stellen aurikulärer Elektroden (AD+, AD-; AD-, AD+) sind.

7. Vorrichtung nach Anspruch 1, in welcher die gemeinsame Stelle eine proximale Elektrode (18) einer Sonde (16) ist.

8. Vorrichtung nach Anspruch 1, in welcher die zwei eigenen Stellen eine proximale Elektrode (12) und eine distale Elektrode (14) einer gleichen Sonde (10) sind.

9. Vorrichtung nach Anspruch 1, in welcher die der Injektion und dem Aufnehmen gemeinsame Stelle eine proximale Elektrode (18) einer aurikularen rechten Sonde (16) ist, die der Injektion eigene Stelle eine distale Elektrode (14) einer ventrikulären rechten Sonde (10) ist und die dem Aufnehmen eigene Stelle eine proximale Elektrode (12) dieser gleichen ventrikulären rechten Sonde (10) ist.

## Claims

1. An active implantable medical device, in particular a pacemaker, defibrillator and/or cardiovertor and/or multisite device, in which electrodes are placed in a plurality of respective distinct sites comprising at least one ventricular site and one atrial site on the same side of the heart, said electrodes being connected to means for sensing cardiac signals, for detecting a depolarisation potential, as well as stimulation means to apply stimulation pulses to at least certain ones of the said sites,
said device further comprising means for evaluating the cardiac flow upon transvalvular bioimpedance measurement, said means operating by injecting of a current between an atrial site and a ventricular site and sensing a differential potential between an atrial site and a ventricular site,
said device being **characterised in that** the transvalvular impedance measurement arrangement is a tripolar arrangement, with one site common to injection and sensing, one site dedicated to injection, and one site dedicated to sensing, the common site (AD+) being located in one of the cavities, and the two dedicated sites (VD+, VD-) being located in the other cavity.

2. The device of claim 1, said device being a multisite device further comprising means for varying the inter-ventricular delay of the application of the respective stimulation pulses on the right and left ventricles, said means operating in response to the measured transvalvular impedance in a direction that results in an improvement of the cardiac flow.

3. The device of claim 1, further comprising means for varying the frequency of application of the stimulation pulses, said means operating in response to the measured transvalvular impedance in a direction that results in an improvement of the cardiac flow

4. The device of claim 1, further comprising means for varying the atrio-ventricular delay in the application of the stimulation pulses, said means operating in response to the measured transvalvular impedance in a direction that results in an improvement of the cardiac flow

5. The device of claim 1, wherein the common site is an atrial electrode site (AD+, AD-) and the two dedicated sites are ventricular electrode sites (VD+, VD- ; VD-, VD+).

6. The device of claim 1, wherein the common site is a ventricular electrode site (VD+ ; VD-) and the two dedicated sites are atrial electrode sites (AD+, AD- ; AD-, AD+).

7. The device of claim 1, wherein the common site is a proximal electrode (18) of a lead (16).

8. The device of claim 1, wherein the two dedicated sites are a proximal electrode (12) and a distal electrode (14) of a same lead (10).

9. The device of claim 1, wherein the site common to injection and sensing is a proximal electrode (18) of a right atrial lead (16), the site dedicated to injection is a distal electrode (14) of a right ventricular lead (10), and the site dedicated to sensing is a proximal electrode (12) of the same right ventricular lead (10).
